(19) Europäisches Patentamt
European Patent Office
Office européen des brevets

(11) **EP 4 393 543 A1**

(12) **EUROPEAN PATENT APPLICATION**

(43) Date of publication:
**03.07.2024 Bulletin 2024/27**

(21) Application number: **23217650.3**

(22) Date of filing: **18.12.2023**

(51) International Patent Classification (IPC):
***A61N 5/10*** *(2006.01)*

(52) Cooperative Patent Classification (CPC):
**A61N 5/1077; A61N 5/1049;** A61N 2005/1055

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2022 GB 202219835**

(71) Applicant: **Elekta Limited**
**Crawley, Sussex**
**RH10 9RR (GB)**

(72) Inventors:
• **FREEDMAN, Joshua**
  **Crawley, RH10 9RR (GB)**
• **PITMAN, Samantha**
  **Crawley, RH10 9RR (GB)**

(74) Representative: **Beal, James Michael et al**
**Kilburn & Strode LLP**
**Lacon London**
**84 Theobalds Road**
**London WC1X 8NL (GB)**

(54) **OPTIMISED RADIOFREQUENCY CONTROL IN RADIOTHERAPY**

(57) A radiotherapy device, a computer-implemented method and a computer-readable medium are provided. The radiotherapy device comprises an accelerating structure, a magnetron and a controller communicatively coupled to the accelerating structure and the magnetron. The controller is configured to determine a predicted resonant frequency change of the accelerating structure based on an predicted operation of the radiotherapy device. The controller is further configured to adjust an output frequency of the magnetron by the predicted resonant frequency change.

Fig. 6

**Description**

This disclosure relates to radiotherapy, and in particular to optimising automatic radiofrequency control in radiotherapy.

Background

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises a gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

In radiotherapy treatment, it is desirable to deliver a prescribed dose of radiation to a target region of a subject and to limit irradiation of other parts of the subject, i.e. of healthy tissue. Motion of the subject can cause a decreased dose to be applied to the target region and/or an increased dose to be applied to the healthy tissue. To address this, known techniques include monitoring a location of the subject and gating the treatment beam such that radiation is applied only when the subject (i.e. the target region within the subject) is in a desired location and not when the subject/target region is in a suboptimal location. Doing so ensures that the dose actually delivered to the target and surrounding healthy tissue best corresponds to the intended treatment plan typically defined on a static planning image with a nomotion assumption.

There are various physiological motions that can contribute to a total motion of a subject. Discrete, gross or large-scale movements of a subject may include shifting position, coughing or sneezing. The subject may also undergo cyclical, physiological movement. For example, the subject may undergo respiratory motion due to their breathing cycle. The subject may also undergo cardiac motion based on beating of their heart. The subject may also undergo non-periodic (e.g. drifting) motion. For example, bladder filling may cause the prostate to drift in position. These motions can alter the location of a subject and/or of a tumour in a time-dependent manner relative to the respective location of the subject and/or of the tumour at the start of the radiotherapy treatment.

Application of radiation by the radiation source in some time periods but not in others may be achieved by gating of a radiation beam emitted by the radiation source. The radiation source may comprise an electron source and a radiofrequency (RF) field source. The electron source may provide a source of electrons which generate a radiation dose to be delivered to the subject, for example by impacting a target. The RF field source may electromagnetically accelerate the electrons to a desired velocity suitable for providing the radiation dose. The radiation source may be gated by controlling the electron source to be on or off and/or by controlling the RF field source to be on or off. In this manner, application of a radiation dose by the radiation source can be controlled according to desired parameters.

When the beam is gated by controlling the electron source to be off, a reduced number of electrons may still be released and accelerated. This is known as dark current, and a dark current-related dose can be applied as a result. This is undesirable because it may cause the dose applied to parts of the subject to differ from the dose specified in a treatment plan. There is a need for improved techniques to reduce the undesired dose originating from dark current while preventing damage to or deterioration of components of a beam generation system. Thus, there is a need for a more reliable beam generation system.

The present invention seeks to address these and other disadvantages encountered in the prior art.

Summary

An invention is set out in the independent claims.

According to an aspect, there is provided a radiotherapy device comprising: an accelerating structure; a magnetron; and a controller communicatively coupled to the accelerating structure and the magnetron, the controller being configured to: determine a predicted resonant frequency change of the accelerating structure based on a predicted operation of the radiotherapy device; and adjust an output frequency of the magnetron by the predicted resonant frequency change.

According to a further aspect, there is provided a computer-implemented method comprising: determining a predicted resonant frequency change of an accelerating structure of a radiotherapy device based on a predicted operation of the radiotherapy device; and adjusting an output frequency of a magnetron of the radiotherapy device by the predicted resonant frequency change.

According to a further aspect, there is provided a computer-readable medium storing instructions which, when executed by a processor, cause the processor to perform the method above.

Brief Description of the Drawings

Specific embodiments are now described, by way of example only, with reference to the drawings, in which:

Figure 1 depicts a radiotherapy device or apparatus according to the present disclosure;
Figure 2 depicts example gated delivery according to the present disclosure;
Figure 3 depicts an example beam generation system according to the present disclosure;
Figure 4 depicts an example of resonant frequency change according to the present disclosure;
Figure 5 depicts an example relationship between resonant frequency change and beam delivery states according to the present disclosure;
Figure 6 depicts a computer-implemented method according to the present disclosure;
Figure 7 depicts a block diagram of one implementation of a radiotherapy system according to the present disclosure;
Figure 8 depicts a computer program product according to the present disclosure.

Detailed Description

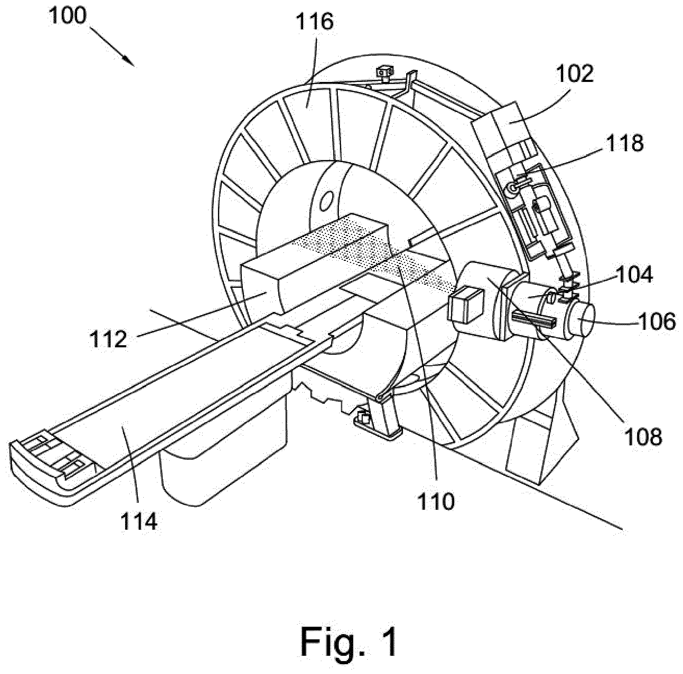

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in Figure 1 is in accordance with the present disclosure and is suitable for use with the disclosed systems and apparatuses. While the device in Figure 1 is an MR-linac, the implementations of the present disclosure may be any radiotherapy device, for example a linac device.

The device 100 depicted in Figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in Figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller. As used herein, a controller may also be referred to as a control device.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move to different positions including a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by one or more subject support surface actuators, which may be described as an actuation mechanism. The actuation mechanism is configured to move the subject support surface at least in a direction parallel to, and defined by, the central axis of the bore. The terms subject and patient are used interchangeably herein such that the patient support surface can also be described as a subject support surface. The subject support surface may also be referred to as a moveable or adjustable couch or table or as a patient couch or subject couch.

The radiotherapy apparatus / device depicted in Figure 1 also comprises MR imaging apparatus 112. The MR imaging apparatus 112 is configured to obtain images of a subject positioned, i.e. located, on the patient support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller. While the discussion herein may focus on MR imaging by way of example, alternatively or in addition to MR imaging, one or more other imaging techniques, modalities, sensors or detectors may be used, such as CT/X-ray, PET, optical imaging/cameras, infra-red imaging, ultra-sound imaging or time-of-flight techniques. Any one or more of these may be used to generate images of the subject and/or to determine the position of the target.

The controller is a computer, processor, or other processing apparatus. The controller may be formed by several discrete processors; for example, the controller may comprise a processor for each of the various individual components of the radiotherapy device as described herein. The controller is communicatively coupled to a memory, e.g. a computer readable medium. The controller may be communicatively coupled to one, multiple or all of the various individual components of the radiotherapy device as described herein. As used herein, the controller may also be referred to as a control device.

The linac device also comprises several other components and systems as will be understood by the skilled person. For example, in order to ensure the linac does not leak radiation, appropriate shielding is also provided.

The radiotherapy device and/or the control device may be configured to perform any of the method steps presently disclosed and may comprise computer executable instructions which, when executed by a processor cause the processor to perform any of the method steps presently disclosed, or when executed by the control device cause the control device to perform any of the method steps presently disclosed, or when executed by the radiotherapy device cause the radiotherapy device to perform any of the method steps presently disclosed. Any of the steps that the radiotherapy device and/or the control device is configured to perform may be considered as method steps of the present disclosure and may be embodied in computer executable instructions for execution by a processor. A computer-readable medium may comprise the above-described computer executable instructions.

In gated treatments, the treatment beam may be held (turned off) and released (turned on) dependent on whether a target is respectively outside or inside a pre-defined volume. The target is an anatomical location within the subject which it is intended to apply a dose to, i.e. a tumour. The pre-defined volume is known as the gating window. When the target is inside the gating window, the beam is turned on; when the target is outside the gating window, the beam is turned off.

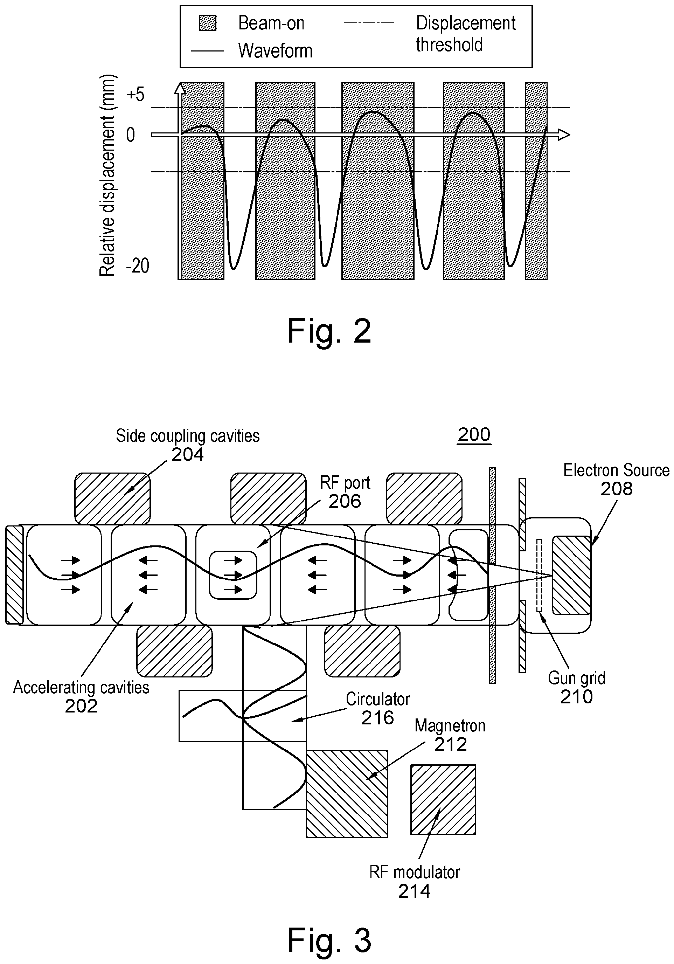

Figure 2 depicts an example gated delivery. The displacement of the target from a reference position (y-axis) is plotted as a function of time (x-axis). The displacement of the target in this example oscillates as a function of time due to periodic movement of the subject, e.g. due to respiration. The gating window is defined by upper and lower displacement thresholds. When the target is within the gating window, it is in a suitable location for delivering dose to the target and therefore the beam is turned on. This is depicted in Figure 2 by the shaded portions. When the target moves outside of the gating window (e.g. due to the subject exhaling), the beam is turned off (non-shaded portions in Figure 2).

During a gated delivery, the position and/or movement of the subject or a part thereof may be determined by one or more sensors. The one or more sensors may comprise any suitable device configured to determine, monitor or measure a position of the subject or a part of the subject, for example a target such as a tumour. The position sensor may be an imaging device, such as an MR imaging device. The (MR) imaging device may determine three-dimensional spatial information for the subject/target. While the discussion herein may focus on MR imaging by way of example, alternatively or in addition to MR imaging, one or more other imaging techniques, modalities, sensors or detectors may be used, such as CT/X-ray, PET, optical imaging/cameras, infra-red imaging, ultra-sound imaging or time-of-flight techniques. Any one or more of these may be used to determine the position of the target.

As the skilled person will be familiar with, MR imaging is an imaging technique which involves placing a subject in a strong magnetic field which aligns the magnetic moments of protons in the subject to produce a net magnetization. Irradiating the subject with RF pulses of a particular resonant frequency tips the net magnetization of these protons by a flip-angle $\alpha°$ into a plane perpendicular to the strong magnetic field. Immediately after the RF pulse is completed, the tipped net magnetization of these protons realigns with the strong magnetic field. The changing magnetic flux generated during realignment induces a voltage in a coil. This is measured and analysed to provide information on the distribution of different tissues within the subject.

A treatment plan for a subject may comprise information specifying the radiotherapy machine parameters to be used for treating the subject. This is based on the subject being disposed in a particular position within or relative to the radiotherapy device. For example, this may be based on one or more pretreatment or template images of the subject. These machine parameters may be optimised for delivering a particular radiative dose to the anatomical position of the target (tumour) within the subject and avoiding delivering substantial dose to healthy tissue within the subject. However, during a radiotherapy treatment session, the subject or parts of the subject may move, for example due to breathing, cardiac motion, coughing, twitching, etc. Imaging the subject during the treatment session can provide a real-time, or close to real-time, check on the position of the subject or parts of the subject, which can be used to determine whether/when the subject is in a suitable position for delivering radiation to the target.

An algorithm may be used to determine the position of the subject or parts thereof from obtained images. As used herein, this will be referred to as an anatomical position monitoring (APM) algorithm. By way of illustration, the discussion below will focus on an APM algorithm using 2D MR images, though it will be appreciated that other imaging modalities and techniques may be used to monitor the position of the subject or parts thereof. During a gated delivery, 2D MR images of the subject may be acquired for motion-monitoring purposes. Based on these images, an APM target trajectory signal may be determined, which may estimate the positions of anatomical parts of the subject as a function of time relative to one or more static template images, for example by comparing the positions of the anatomical features in the different images. Various methods may be used for estimating the target trajectory signal on 2D MR images. One possible embodiment includes the use of optimization-based registration of each image in the 2D MR image series to a template (reference) image. Another possible embodiment includes the use of machine learning regression methods.

The APM target trajectory signal is affected by system latency, i.e. time-delay, between anatomical positions of the subject and system responses to those positions. For example, for MR imaging, this system latency may include contributions from the duration or frequency of acquisition, the duration of reconstruction of an image, the preparation of the image for transmission, the transmission of the image to a motion manager, the determination of a decision based on the image and reference data/predetermined conditions, and the response time of the radiotherapy device to alter or interrupt the treatment. These contributions entail that the response to the target being in a particular location is based on the location of the target at some point in the past, i.e. in the past by a time period referred to as the system latency. If the target moves during this time period, the use of such out of date information may lead to incorrect or inaccurate responses, which can cause dosimetric errors.

One approach to dealing with such system latency is to use a prediction model, for example based on linear regression filters. The prediction model can be trained on the target waveform calculated from previously acquired data and then applied to infer the waveform at a future time, thereby effectively reducing the system latency by looking ahead to how the anatomy of the subject is expected to move at subsequent timepoints. This prediction method can be used to compensate the APM signal for system latencies.

It will be appreciated that the periodic or pseudo-periodic motion of the subject depicted in Figure 2 aids the prediction of future motion. This may be due to a regular physiological pattern of the subject, e.g. a respiratory or cardiac cycle. The delivery state of the beam may be described as beam on or beam off. Because the beam on/beam off state depends on when the target is within the gating window, and because the target is within the gating window at regular intervals, the future time periods for which the beam will be on or off can be predicted.

Figure 3 depicts an example of a beam generation system 200 of a radiotherapy device according to the present disclosure. The beam generation system 200 may comprise a linac (linear accelerator) or part of a linac, which may comprise part of a radiotherapy device as described herein. The beam generation system 200 is configured to generate a beam for delivering radiotherapy. In the beam generation system 200, electrons are injected into an accelerating structure, in which they are accelerated using RF power. The accelerated electrons may strike a tungsten alloy target, which produces the beam. The accelerating structure may be referred to as a waveguide, an accelerating waveguide, a series of accelerating cavities, or a series of waveguide cells.

The beam generation system 200 may comprise an electron source 208, for example an electron gun, configured to produce electrons. These electrons may pass through a gun grid 210. Gating may be enabled by pulsing the voltage applied to the gun grid 210 in order to control the attraction/repulsion of the negatively charged electrons. The electrons are accelerated in an accelerating structure. The accelerating structure may comprise a waveguide having waveguide cells. The accelerating structure may comprise a series of interconnected accelerating cavities 202. The accelerating structure may comprise side coupling cavities 204 and one or more RF ports 206. A magnetron 212 is configured to generate RF power, and the magnetron 212 may be driven by pulsing of an RF modulator 214. The RF power generated by the magnetron 212 may pass through a circulator 216 and be provided to the accelerating structure, for example through the RF port(s) 206. The circulator 216 is configured to reject reflected RF power. The frequency of the RF power generated by the magnetron 212 should match the resonant frequency of the accelerating structure in order that maximum power is provided to the accelerator and minimum power is reflected out via the circulator 216.

Gating is enabled by holding or releasing the beam by pulsing the voltage of the gun grid 210. Independently of this, the RF modulator 214 may be configured to continue pulsing at a pulse repetition frequency (PRF). In other words, the PRF may be maintained at its nominal value whether or not the beam is gated via the voltage applied to the gun grid 210. Therefore, a standing RF wave may be continuously maintained in the accelerating structure during a gated delivery, i.e. in both the beam on and beam off states. In Figure 3, this standing wave is depicted with groups of three horizontal arrows.

Maintaining the nominal PRF throughout a gated delivery can lead to undesirable dark current-related dose. This dark current can originate from electrons arising from the walls of the accelerating structure and/or from electrons that have escaped past the gun grid 210. In order to mitigate the undesirable dose derived from the dark current, the PRF may be adjusted during the gated delivery. During beam hold (when the beam is off), the PRF could be reduced from its nominal value of 275 Hz to a lower value (e.g. 100 Hz) or could be completely removed (i.e. operate at 0 Hz). However, lowering the PRF results in rapid cooling of the accelerating structure. This is because the water cooling system configured to cool the accelerating structure continues to act to lower the temperature of the accelerating structure, but the heating caused by the RF power is reduced or removed.

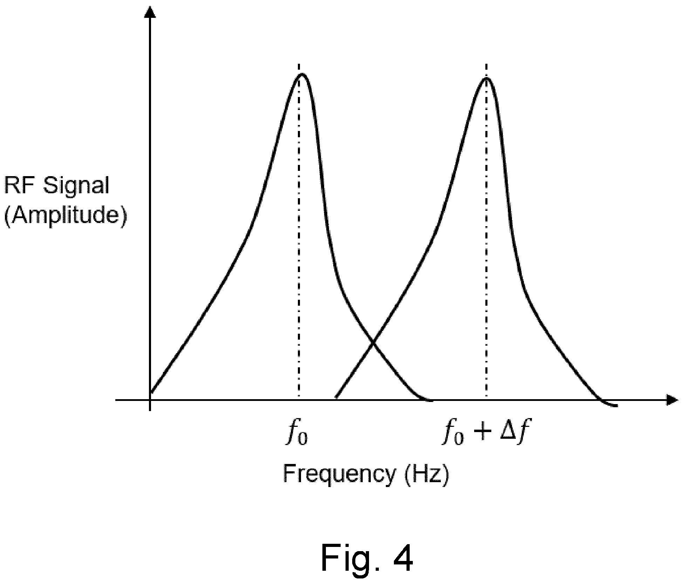

As the accelerating structure cools it shrinks, which leads to an increase in the resonant frequency of the accelerating structure. This increase in resonant frequency from $f_0$ to $f_0 + \Delta f$ is depicted in Figure 4, which shows RF amplitude (y-axis) plotted against frequency (x-axis). A change in temperature of the accelerating structure causes its resonant frequency to change by $\Delta f$.

In normal operation, the resonant frequency of the accelerating structure drifts up and down. An automatic frequency controller (AFC) is configured to tune the output frequency of the magnetron 212 to match it to the current resonant frequency of the accelerating structure. This minimises the amount of RF power rejected from the accelerating structure and returned through the circulator 216. The AFC is configured to compare the reflected RF signal to the forward RF signal and produce a voltage proportional to the error (i.e. the difference). This voltage is used to adjust the output frequency of the magnetron 212, e.g. by driving a motion that turns a tuner on the side of the magnetron 212. In other words, the AFC is configured to detect phase shift at the circulator 216 and to drive a servo tuner drive motor to tune the output frequency of the magnetron 212.

The AFC has a limited range of output frequencies which it can tune to, outside of which it may not be able to adjust the output frequency of the magnetron 212 to a suitable value. The output frequency of the magnetron 212 differing from the resonant frequency of the accelerating structure results in low dose and can lead to beam termination. In addition, stopping the magnetron 212 from pulsing during beam holds, to prevent dark current as described above, removes the input signal to the AFC such that it cannot track the time-varying resonant frequency of the accelerating structure. When the beam is turned back on again, this can lead to a delay between the beam on signal and the linac producing a beam at the correct dose rate. The AFC may then self-recover after a delay, but this would still lead to radiation being delivered off-resonance during that delay period. This can lead to dosimetric errors. In addition, repeatedly operating the magnetron 212 off-resonance may lead to reliability issues and a shortened lifespan of the magnetron 212.

It will be appreciated based on the above discussion that there is a need for improved control of a beam generation system. In particular, it would be desirable to provide improved control of a beam generation system for gated deliveries. It would also be desirable to improve the accuracy of the dose delivered during a radiotherapy treatment. It would also be desirable to improve the reliability of a beam generation system.

The current disclosure provides a prediction-based tuning algorithm to optimise the output frequency of the magnetron 212 where the PRF is reduced or removed during the beam-hold state in gated deliveries. This enables the dose originating from dark current to be reduced while matching the output frequency of the magnetron 212 to the time-varying resonant frequency of the accelerating structure.

As explained above, a prediction model can be used to predict the location of a target at future times based on measurements of target location at previous times. The prediction algorithm may therefore be said to determine the target position as a function of time, denoted $P(t)$. A gating window may be defined by the upper and lower bounds $[G_{lower}, G_{upper}]$. This gating window may be set by a user, for example based on a treatment plan. A binary gating decision signal $G(t)$ at time $t$ can then be defined as:

$$G_{lower} < P(t) < G_{upper}; \ G(t) = 1$$

$$P(t) < G_{lower}; \ G(t) = 0$$

$$P(t) > G_{upper}; \ G(t) = 0$$

A value of 1 for $G(t)$ indicates that the beam is on, while a value of 0 indicates that the beam is off.

The length of a beam hold, i.e. the time for which the beam is off, may be denoted $\Delta t_{BH}$. The resonant frequency change $\Delta f$ of the accelerating structure is dependent on the duration $\Delta t_{BH}$. The longer the beam is off for, the more the accelerating structure cools down and the larger the increase in the resonant frequency. This relationship is highly predictable and almost entirely linear. It may be experimentally measured for each radiotherapy device individually. Alternatively, the relationship may be determined in an averaged manner across multiple radiotherapy devices of the same type.

By way of example, once the RF is stopped, the resonant frequency $f_0$ of the accelerating structure may increase by 20 kHz/s. For a beam hold time $\Delta t_{BH} = 2$ seconds, the output frequency of the magnetron 212 must therefore be increased by 40 kHz before the beam is turned on again.

While the resonant frequency change $\Delta f$ increases with the beam hold time $\Delta t_{BH}$ due to cooling of the accelerating structure, it decreases with the time the beam is on or resumed or released, denoted $\Delta t_R$, due to heating of the accelerating structure. In general, this relationship can be expressed as:

$$\Delta f \propto \Delta t_{BH} - \Delta t_R$$

This follows from the assumption that temperature changes associated with $\Delta t_{BH}$ and $\Delta t_R$ result in increases and decreases to $\Delta f$, respectively. Using the definition of $G(t)$ above, these times can be expressed as:

$$\Delta t_{BH} = \sum_{t=T_0}^{T_1} \big(1 - G(t)\big); \quad \Delta t_R = \sum_{t=T_0}^{T_1} \big(G(t)\big)$$

Substituting these into the expression for the resonant frequency change $\Delta f$ yields the relationship:

$$\Delta f \propto \sum_{t=T_0}^{T_1} \left(1 - 2G(t)\right)$$

In the above expressions, the term $T_0$ refers to the previous time at which the AFC tuned the structure to the resonant frequency. The time $T_1$ is the time at which the frequency correction is to be estimated. In the above example: $T_0 = 0$, $T_1 = 2$ seconds, $\Delta t_R = 0$ and $\Delta t_{BH} = 2$ seconds. Beam-hold/Beam-hold release events closer to $T_1$ are expected to contribute more to $\Delta f$ than those near $T_0$. This is because thermal drifts associated with earlier time-points (i.e., closer to $T_0$) should be damped over time. A weighting coefficient $a(t)$ may be introduced to account for the thermal drift:

$$\Delta f(t, T_0, T_1) \propto \sum_{t=T_0}^{T_1} \left(1 - 2G(t)\right)a(t)$$

As such, the contribution of the different beam on and beam off times to the frequency change may be weighted by the time-dependent weighting factor $a(t)$, which may increase closer to the time $T_1$ at which the frequency correction is to be estimated for, i.e. to be applied.

For simplicity purposes, two choices are made: $T_0 = 0$, $T_1 = T$; where $\Delta t_{BH} + \Delta t_R = T$. The following boundary conditions may be asserted to reflect the thermal drift behaviour, i.e. that beam-hold states closer in time to $T$ contribute a higher weighting to $\Delta f$.

$$t \to 0; \; a(t) \to 0$$

$$t \to T; \; a(t) \to 1$$

To achieve this, the weighting, $a(t) = e^{-\alpha(T-t)}$ is hypothesized, where $\alpha$ represents a scaling term used to tune the damping extent. It will be appreciated that other weightings may be applied. Substituting in the weighting yields the expression:

$$\Delta f(t, \alpha, T) \propto \sum_{t=0}^{T} \left(1 - 2G(t)\right) e^{-\alpha(T-t)}$$

In the integral limit this becomes:

$$\Delta f(t, \alpha, T) \propto \int_{t=T_0}^{T_1} \left(1 - 2G(t)\right) e^{-\alpha(T_1-t)} \, dt$$

This derived equation describes the relationship between the change in resonant frequency $\Delta f$ and the binary gating decision signal $G(t)$. This may be solved as set out below:

$$\Delta f(t, \alpha, T) \propto \int_{t=0}^{T} \left(1 - 2G(t)\right) e^{-\alpha(T-t)} \, dt$$

$$\Delta f(t, \alpha, T) \propto e^{-\alpha T} \int_{t=0}^{T} e^{\alpha t} - 2e^{-\alpha T} \int_{t=0}^{T} G(t) e^{\alpha t} \, dt$$

Let: $J1 = e^{-\alpha T} \int_{t=0}^{T} e^{\alpha t}$; $J2 = 2e^{-\alpha T} \int_{t=0}^{T} G(t) e^{\alpha t} dt$; such that $\Delta f(t, \alpha, T) \propto J1 - J2$

The integral $J1$ may be solved as:

$$J1 = \frac{1}{\alpha}(1 - e^{-\alpha T})$$

The integral $J2$ may be solved using integration by parts:

$$\int u\, dv = uv - \int v\, du$$

$$u = e^{\alpha t}; \quad du = \alpha e^{\alpha t};$$

$$dv = G(t); \quad v = \int G(t)\, dt$$

The integral of $v = \int G(t)\, dt$ may be written as: $v = \gamma t$, where $\gamma = \dfrac{\Delta t_R}{T}$.

In other words, integrating over $G(t)$ results in the accumulated time of the breath-hold release states. Then:

$$J2 = 2e^{-\alpha T} \int_{t=0}^{T} G(t) e^{\alpha t}\, dt = e^{\alpha t} \gamma t \big|_0^T - \int_{t=0}^{T} \gamma t \alpha e^{\alpha t}\, dt$$

$$J2 = 2e^{-\alpha T}\left( e^{\alpha t} \gamma t \big|_0^T - \left(\alpha\gamma \left[\frac{e^{\alpha t}(\alpha t - 1)}{\alpha^2}\right]\right)\big|_0^T \right)$$

$$J2 = -\frac{2}{\alpha}\left(\frac{\Delta t_R}{T}\right)(1 - e^{-\alpha T})$$

Substituting $J1$ and $J2$ and further simplification leads to:

$$\Delta f(t, \alpha, T) \propto \frac{1}{\alpha}\left(1 - 2\left(\frac{\Delta t_R}{T}\right)\right)(1 - e^{-\alpha T})$$

Alternatively, the characteristic equation may be written as:

$$\Delta f(t, \alpha, T) = \frac{k}{\alpha}\left(1 - 2\left(\frac{\Delta t_R}{T}\right)\right)(1 - e^{-\alpha T})$$

Where k is a proportionality constant, which may be determined experimentally. The result may be verified in the short time limit $T \to 0$, using a Taylor expansion for $e^{-\alpha T}$:

$$\Delta f(\alpha, T(T_0, T_1)) \propto \frac{1}{\alpha}\left(1 - 2\left(\frac{\Delta t_R}{T}\right)\right)(1 - (1 - \alpha T))$$

$$\Delta f\big(\alpha, T(T_0, T_1)\big) \propto T - 2\Delta t_R$$

$$\Delta f \propto \Delta t_{BH} - \Delta t_R$$

When $T$ is short, the behaviour is linear but becomes increasingly non-linear as the time $T$ is increased.

In some implementations, the derived dependence of $\Delta f$ on $\Delta t_{BH}$ and $\Delta t_R$ may be used to adjust the resonant frequency for both beam off and beam on times. In alternative implementations, the derived dependence may be used to adjust the resonant frequency for the beam off times, and the AFC may be used to adjust the resonant frequency during a beam on time. If the PRF is completely removed during the beam off times, the AFC will not be able to be utilised in the beam off state because the input signal to the AFC would be removed, as described above. The previously stated frequency shift relationship then becomes:

$$\Delta f \propto \Delta t_{BH}$$

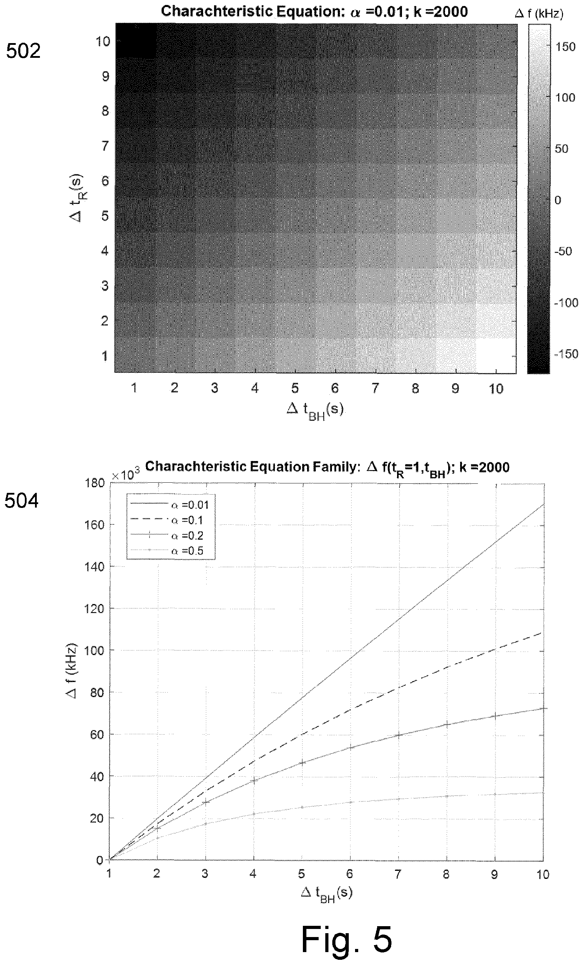

The derived expression reduces to $\Delta f \propto \Delta t_{BH}$ when $T$ is short, which is the case if the system is tuned in near real-time. The relationship between $\Delta f$ and $\Delta t_{BH}$ (and $\Delta t_R$, k and $\alpha$) may be determined empirically for a particular radiotherapy device or in an averaged manner over multiple radiotherapy devices. Alternatively, or in addition, the relationship may be determined based on simulations of the beam generation system. One example simulation of the relationship between $\Delta f$, $\Delta t_{BH}$ and $\Delta t_R$ is depicted in Figure 5. In the top part of Figure 5 (labelled 502), $\alpha$ was set as 0.01 and the proportionality constant k was chosen as 2000 Hz. Increasing $\Delta t_{BH}$ increases $\Delta f$, while increasing $\Delta t_R$ lowers $\Delta f$. As shown in the bottom part of Figure 5 (labelled 504), increasing $\alpha$ values result in increased non-linearity. This demonstrates that a wide range of thermal drift effects may be modelled by the characteristic equation. In addition, it can be observed from 504 that, when $\Delta t_{BH}$ and $\Delta t_R$ are small ($\Delta t_R$ was set to 1), $\Delta f$ is approximately linear. When $\Delta t_{BH}$ increases, the relationship between $\Delta f$ and $\Delta t_{BH}$ becomes increasingly non-linear.

Since the prediction algorithm determines a prediction for the target locations at future times, this provides a prediction for when the target will be within the gating window at future times and when it will be outside. Since the beam is held when the target is outside the gating window and resumed when it is inside, this can be used to determine the values of $\Delta t_{BH}$ and $\Delta t_R$ in an upcoming period. Using these, the value of $\Delta f$ may be determined/calculated/predicted for an upcoming time period.

The value $\Delta f$ may be determined from the derived equation above. The value $\Delta f$ may above based on 2D interpolation, for example of a graph of the form depicted in Figure 5, or of a corresponding dataset. The value $\Delta f$ may be determined from a look-up table. The value $\Delta f$ may be determined from an equation of the form $\Delta f = A\Delta t_{BH} + B\Delta t_R$, where $A$ is a (positive) coefficient, e.g. a first constant value, indicating the dependence of the frequency change on the beam off time, and $B$ is a (negative) coefficient, e.g. a second constant value, indicating the dependence of the frequency change on the beam on time. This equation may be determined based on measurements of the radiotherapy device (or multiple radiotherapy devices of the same type), and/or based on simulations of the beam generation system.

The output frequency of the magnetron 212 may be adjusted by $\Delta f$. This may be performed by the controller, by the AFC, or by the RF modulator 214. This enables this output frequency to stay closely matched to the resonant frequency of the accelerating structure even when the PRF is zero and there is no input signal to the AFC. In other words, the output frequency is adjusted by an appropriate amount to match the predicted resonant frequency of the accelerating structure at the time when the beam is predicted to be turned back on. The adjustment may be made while the beam is turned off, e.g. just before the beam is predicted to be turned back on.

These techniques avoid scenarios in which the beam is turned off for some (unknown) time, and, when the beam is turned back on, the magnetron 212 initially provides RF power at a frequency poorly matched to the resonant frequency of the accelerating structure, which will have changed relative to its resonant frequency when the beam was first turned off. It therefore enables operation of the magnetron 212 off-resonance to be avoided. This enables more accurate application of an intended dose and improves the reliability and lifespan of the magnetron 212.

Alternatively or in addition to the techniques described above, the value of $\Delta f$ may be used on-the-fly to verify the AFC RF power error calculation for situations in which the PRF is not zero such that there is in fact an input signal to the AFC. In other words, the value of $\Delta f$ may be used as an independent check on the adjustment to the output frequency of the magnetron 212 to apply. This may improve the reliability and accuracy of the matching of the output frequency of the magnetron 212 to the resonant frequency of the accelerating structure.

While gated deliveries have been focused on in order to describe the techniques of the present disclosure, the present disclosure is not limited thereto. The techniques of the present disclosure may be applied to any deliveries in which the beam is predicted to be on or off at different times. For example, the techniques of the present disclosure may be applied to tracked deliveries. In tracked deliveries, the beam may be held when changing between segments or changing positions of leaves of a (multi-leaf) collimator. This holding of the beam may be known in advance from a treatment plan, and therefore the techniques described herein may be used to adjust the output frequency of the magnetron 212 by an amount matching the predicted change in resonant frequency of the accelerating structure due to the planned beam on and beam off time periods.

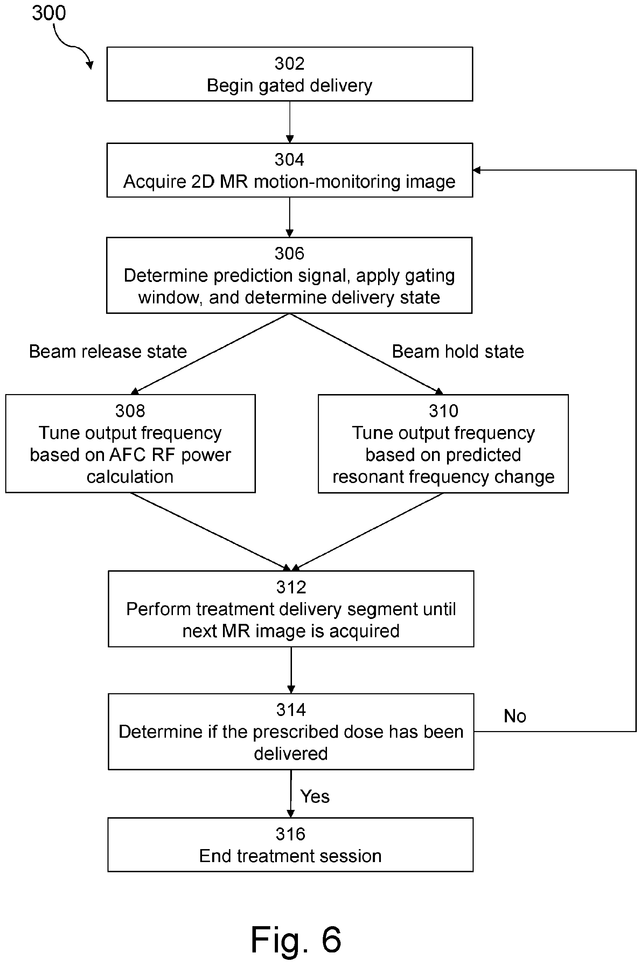

Figure 6 shows a computer-implemented method 300 which is an example clinical implementation of the prediction-based tuning method according to the present disclosure. The method may be performed, for example, by a radiotherapy device, a controller of a radiotherapy device, or a controller/computer communicatively coupled to a radiotherapy device.

In a step 302, a gated delivery may be begun. The gated delivery may comprise a radiation source of the radiotherapy device applying a dose of radiation to a target based on a treatment plan. The beam may be gated on when the target is within a gating window and be gated off when the target is outside the gating window.

In a step 304, a 2D MR motion-monitoring image may be acquired. The image may be obtained by an MR imaging apparatus of the radiotherapy device. In some examples, other forms of MR image or images obtained using other imaging modalities may be acquired and used in place of the 2D MR motion-monitoring image.

In a step 306, a prediction signal may be determined, a gating window may be applied, and a delivery state of the beam may be determined. The prediction signal may be determined based on one or more 2D MR motion-monitoring images. The position of the target at previous timepoints may be used to predict its position at future timepoints based on periodic or pseudo-periodic motion of the target/subject. The delivery state of the beam may be determined to be beam on for time periods for which the prediction signal is within the gating window, and beam off for time periods for which the prediction signal is not within the gating window.

If the delivery state is beam on/beam release, the method continues to step 308. In step 308, the output frequency of the magnetron 212 may be tuned based on an AFC RF power calculation. This may be based on an error signal describing the RF power reflected from the accelerating structure, as described above.

If the delivery state is beam off/beam hold, the method continues to step 310. In step 310, the output frequency of the magnetron 212 may be tuned based on the predicted resonant frequency change of the accelerating structure according to the techniques described herein. In some examples, the output frequency of the magnetron 212 may also be tuned based on the predicted resonant frequency change of the accelerating structure according to the techniques described herein in step 308, alternatively or in addition to the tuning based on the AFC RF power calculation.

In a step 312, a treatment delivery segment may be performed until the next MR image is acquired. In other words, a treatment session may continue with the magnetron 212 providing an adjusted output frequency according to step 308/310.

In a step 314, it may be determined if the prescribed dose has been delivered. For example, the dose delivered may be measured and compared to the dose prescribed in a treatment plan. If the prescribed dose has not been delivered, the method may return to step 304. If the prescribed dose has been delivered, the method may continue to step 316, at which the treatment session is ended.

While the methods disclosed herein are presented in a certain sequential order, this should not be taken to limit the methods to the orders presented. One or more of the method steps may be omitted or rearranged. The various steps may be performed in different orders. Various steps may be performed at the same time or substantially the same time. Herein, references to events occurring substantially at the same time may refer to events at least partially overlapping in time and/or events occurring at the same time within measurement uncertainties.

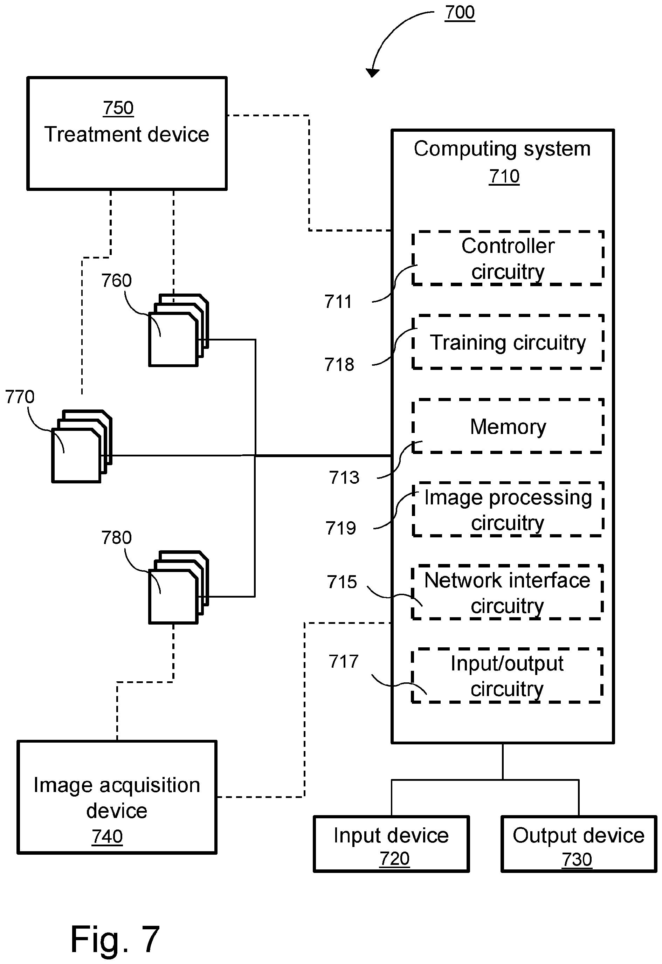

Figure 7 illustrates a block diagram of one implementation of a radiotherapy system 700. The radiotherapy system 700 comprises a computing system 710 within which a set of instructions, for causing the computing system 710 to perform any one or more of the methods discussed herein, may be executed.

The computing system 710 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random access memory (DRAM) such as synchronous DRAM (SDRAM) or Rambus DRAM (RDRAM), etc.). The memory 713 may comprise a static memory (e.g., flash memory, static random access memory (SRAM), etc.), and/or a secondary memory (e.g., a data storage device), which communicate with each other via a bus (not shown).

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 715. The computing system 710 may be communicatively coupled to an input device 720 and/or an output device 730, via input/output circuitry 717. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

In some implementations, computing system 710 includes training circuitry 718. The training circuitry 718 is configured to train a method of determining a predicted resonant frequency change as described herein. For example, training circuitry 718 may train a model for determining a predicted resonant frequency change as described herein. The model may comprise a deep neural network (DNN), such as a convolutional neural network (CNN) and/or recurrent neural network (RNN). Training circuitry 718 may be configured to execute instructions to train a model that can be used to determine a predicted resonant frequency change as described herein. Training circuitry 718 may be configured to access training data and/or testing data from memory 713 or from a remote data source, for example via network interface circuitry 715. In some examples, training data and/or testing data may be obtained from an external component, such as image acquisition device 740 and/or treatment device 750. In some implementations, training circuitry 718 may be used to update, verify and/or maintain the model determining a predicted resonant frequency change as described herein.

In some implementations, the computing system 710 may comprise image processing circuitry 719. Image processing circuitry 619 may be configured to process image data 780 (e.g. images, or imaging data), such as medical images obtained from one or more imaging data sources, a treatment device 750 and/or an image acquisition device 740 as described herein. Image processing circuitry 719 may be configured to process, or pre-process, image data. For example, image processing circuitry 719 may convert received image data into a particular format, size, resolution or the like. In some implementations, image processing circuitry 719 may be combined with controller circuitry 711.

In some implementations, the radiotherapy system 700 may further comprise an image acquisition device 740 and/or a treatment device 750, such as those disclosed herein (e.g. in relation to Figure 1). The image acquisition device 740 and the treatment device 750 may be provided as a single device. In some implementations, treatment device 750 is configured to perform imaging, for example in addition to providing treatment and/or during treatment. The treatment device 750 comprises the main radiation delivery components of the radiotherapy system described herein.

Image acquisition device 740 may be configured to perform positron emission tomography (PET), computed tomography (CT), magnetic resonance imaging (MRI), etc.

Image acquisition device 740 may be configured to output image data 780, which may be accessed by computing system 710. Treatment device 750 may be configured to output treatment data 760, which may be accessed by computing system 710.

Computing system 710 may be configured to access or obtain treatment data 760, planning data 770 and/or image data 780. Treatment data 760 may be obtained from an internal data source (e.g. from memory 713) or from an external data source, such as treatment device 750 or an external database. Planning data 770 may be obtained from memory 713 and/or from an external source, such as a planning database. Planning data 770 may comprise information obtained from one or more of the image acquisition device 740 and the treatment device 750.

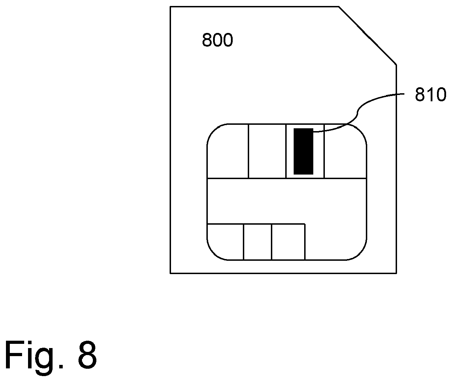

The various methods described above may be implemented by a computer program. The computer program may include computer code (e.g. instructions) 810 arranged to instruct a computer to perform the functions of one or more of the various methods described above. The steps of the methods described above may be performed in any suitable order. The computer program and/or the code 810 for performing such methods may be provided to an apparatus, such as a computer, on one or more computer readable media or, more generally, a computer program product 800)), depicted in Figure 8. The computer readable media may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 810 may also reside, completely or at least partially, within the memory 713 and/or within the controller circuitry 711 during execution thereof by the computing system 710, the memory 713 and the controller circuitry 711 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "predicting", "determining", "adjusting ", "enabling", "maintaining," "identifying," "obtaining," "detecting," "generating," "calculating," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

The disclosure comprises the following items:

1. A radiotherapy device comprising:

an accelerating structure;
a magnetron; and
a controller communicatively coupled to the accelerating structure and the magnetron, the controller being configured to:

determine a predicted resonant frequency change of the accelerating structure based on a predicted operation of the radiotherapy device; and
adjust an output frequency of the magnetron by the predicted resonant frequency change.

2. A radiotherapy device according to item 1, wherein the predicted operation of the radiotherapy device comprises a beam delivery state of the radiotherapy device.

3. A radiotherapy device according to item 2, wherein the beam delivery state comprises a beam off time period.

4. A radiotherapy device according to item 3, wherein the controller is configured to determine the predicted resonant frequency change to increase as a function of the beam off time period.

5. A radiotherapy device according to any of items 2-4, wherein the beam delivery state comprises a beam on time period.

6. A radiotherapy device according to item 5, wherein the controller is configured to determine the predicted resonant frequency change to decrease as a function of the beam on time period.

7. A radiotherapy device according to item 5 or item 6, wherein the radiotherapy device comprises an automatic frequency controller configured to adjust the output frequency of the magnetron during the beam on time period.

8. A radiotherapy device according to any preceding item, wherein the predicted operation of the radiotherapy device comprises a gated radiotherapy treatment.

9. A radiotherapy device according to item 8, wherein the radiotherapy device comprises a sensor configured to determine a position of a part of a subject in the radiotherapy device, and the controller is configured to determine the predicted operation of the radiotherapy device at future timepoints based on the determined position at previous timepoints.

10. A radiotherapy device according to item 9, wherein the controller being configured to determine the predicted operation of the radiotherapy device at future timepoints comprises the controller being configured to predict at which of the future timepoints the part of the subject will be within a spatial window.

11. A radiotherapy device according to item 9 or item 10, wherein the sensor is an MR imaging apparatus.

12. A radiotherapy device according to any preceding item, wherein the controller is configured to determine the predicted resonant frequency change as a function of the predicted operation of the radiotherapy device based on experimental measurements taken on the radiotherapy device.

13. A computer-implemented method comprising:

determining a predicted resonant frequency change of an accelerating structure of a radiotherapy device based on a predicted operation of the radiotherapy device; and
adjusting an output frequency of a magnetron of the radiotherapy device by the predicted resonant frequency change.

14. A computer-implemented method according to item 13, wherein the predicted operation of the radiotherapy device comprises a beam delivery state of the radiotherapy device.

15. A computer-implemented method according to item 14, wherein the beam delivery state comprises a beam off time period.

16. A computer-implemented method according to item 15, comprising determining the predicted resonant frequency change to increase as a function of the beam off time period.

17. A computer-implemented method according to any of items 14-16, wherein the beam delivery state comprises a beam on time period.

18. A computer-implemented method according to item 17, comprising determining the predicted resonant frequency change to decrease as a function of the beam on time period.

19. A computer-implemented method according to item 17 or item 18, comprising adjusting, by an automatic frequency controller of the radiotherapy device, the output frequency of the magnetron during the beam on time period.

20. A computer-implemented method according to of items 13-19, wherein the predicted operation of the radiotherapy device comprises a gated radiotherapy treatment.

21. A computer-implemented method according to item 20, comprising determining, using a sensor of the radiotherapy device, a position of a part of a subject in the radiotherapy device, and determining the predicted operation of the radiotherapy device at future timepoints based on the determined position at previous timepoints.

22. A computer-implemented method according to item 22, wherein determining the predicted operation of the radiotherapy device at future timepoints comprises predicting at which of the future timepoints the part of the subject will be within a spatial window.

23. A computer-implemented method to item 21 or item 22, wherein the sensor is an MR imaging apparatus.

24. A computer-implemented method according to any of items 13-23, comprising determining the predicted resonant frequency change as a function of the predicted operation of the radiotherapy device based on experimental measurements taken on the radiotherapy device.

25. A computer-readable medium storing instructions which, when executed by a processor, cause the processor to perform the method of any of items 13-24.

**Claims**

1. A radiotherapy device comprising:

   an accelerating structure;
   a magnetron; and
   a controller communicatively coupled to the accelerating structure and the magnetron, the controller being configured to:

   determine a predicted resonant frequency change of the accelerating structure based on a predicted operation of the radiotherapy device; and
   adjust an output frequency of the magnetron by the predicted resonant frequency change.

2. A radiotherapy device according to claim 1, wherein the predicted operation of the radiotherapy device comprises a beam delivery state of the radiotherapy device.

3. A radiotherapy device according to claim 2, wherein the beam delivery state comprises a beam off time period.

4. A radiotherapy device according to claim 3, wherein the controller is configured to determine the predicted resonant frequency change to increase as a function of the beam off time period.

5. A radiotherapy device according to any of claims 2-4, wherein the beam delivery state comprises a beam on time period.

6. A radiotherapy device according to claim 5, wherein the controller is configured to determine the predicted resonant frequency change to decrease as a function of the beam on time period.

7. A radiotherapy device according to claim 5 or claim 6, wherein the radiotherapy device comprises an automatic frequency controller configured to adjust the output frequency of the magnetron during the beam on time period.

8. A radiotherapy device according to any preceding claim, wherein the predicted operation of the radiotherapy device comprises a gated radiotherapy treatment.

9. A radiotherapy device according to claim 8, wherein the radiotherapy device comprises a sensor configured to determine a position of a part of a subject in the radiotherapy device, and the controller is configured to determine the predicted operation of the radiotherapy device at future timepoints based on the determined position at previous timepoints.

10. A radiotherapy device according to claim 9, wherein the controller being configured to determine the predicted operation of the radiotherapy device at future timepoints comprises the controller being configured to predict at which of the future timepoints the part of the subject will be within a spatial window.

11. A radiotherapy device according to claim 9 or claim 10, wherein the sensor is an MR imaging apparatus.

12. A radiotherapy device according to any preceding claim, wherein the controller is configured to determine the predicted resonant frequency change as a function of the predicted operation of the radiotherapy device based on experimental measurements taken on the radiotherapy device.

13. A computer-implemented method comprising:

> determining a predicted resonant frequency change of an accelerating structure of a radiotherapy device based on a predicted operation of the radiotherapy device; and
> adjusting an output frequency of a magnetron of the radiotherapy device by the predicted resonant frequency change.

14. A computer-readable medium storing instructions which, when executed by a processor, cause the processor to perform the method of claim 13.

Fig. 1

Fig. 2

Fig. 3

Fig. 4

502

Charachteristic Equation: $\alpha$ =0.01; k =2000

504

Charachteristic Equation Family: $\Delta f(t_R=1, t_{BH})$; k =2000

## Fig. 5

300

302
Begin gated delivery

304
Acquire 2D MR motion-monitoring image

306
Determine prediction signal, apply gating window, and determine delivery state

Beam release state                    Beam hold state

308
Tune output frequency based on AFC RF power calculation

310
Tune output frequency based on predicted resonant frequency change

312
Perform treatment delivery segment until next MR image is acquired

314
Determine if the prescribed dose has been delivered

No

Yes

316
End treatment session

Fig. 6

700

| 750 Treatment device |

| Computing system 710 |

711 — Controller circuitry

718 — Training circuitry

713 — Memory

719 — Image processing circuitry

715 — Network interface circuitry

717 — Input/output circuitry

760

770

780

| Image acquisition device 740 |

| Input device 720 | | Output device 730 |

# Fig. 7

800

810

Fig. 8

Europäisches
Patentamt

European
Patent Office

Office européen
des brevets

# EUROPEAN SEARCH REPORT

Application Number

EP 23 21 7650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/326636 A1 (EATON DOUGLAS W [US] ET AL) 27 December 2012 (2012-12-27) | 1-8, 12-14 | INV. A61N5/10 |
| Y | * paragraphs [0003] – [0007], [0012] – [0023], [0035], [0036], [0060] – [0066] * <br> * figures 1,3,4,10 * | 9-11 | |
| X | KR 2020 0050676 A (KERI KOREA ELECTROTECHNOLOGY RES INST [KR]) 12 May 2020 (2020-05-12) <br> * paragraphs [0028] – [0048] * <br> * figure 1 * | 1,2, 12-14 | |
| X | US 2019/357343 A1 (TURNER JOHN C [US] ET AL) 21 November 2019 (2019-11-21) <br> * paragraphs [0122] – [0143] * <br> * figure 8 * | 1,12-14 | |
| Y | CRIJNS S P M ET AL: "Towards MRI-guided linear accelerator control: gating on an MRI accelerator;Towards MRI-guided linear accelerator control", PHYSICS IN MEDICINE AND BIOLOGY, INSTITUTE OF PHYSICS PUBLISHING, BRISTOL GB, vol. 56, no. 15, 13 July 2011 (2011-07-13) , pages 4815-4825, XP020208295, ISSN: 0031-9155, DOI: 10.1088/0031-9155/56/15/012 * the whole document * | 9-11 | TECHNICAL FIELDS SEARCHED (IPC) <br><br> A61N |
| A | EP 1 317 944 A1 (HITESYS SPA [IT]) 11 June 2003 (2003-06-11) <br> * paragraph [0012] * | 1-14 | |

The present search report has been drawn up for all claims

| Place of search | Date of completion of the search | Examiner |
|---|---|---|
| Munich | 29 April 2024 | Kretschmann, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 7650

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2024

| Patent document cited in search report | | Publication date | Patent family member(s) | | Publication date |
|---|---|---|---|---|---|
| US 2012326636 | A1 | 27-12-2012 | NONE | | |
| KR 20200050676 | A | 12-05-2020 | NONE | | |
| US 2019357343 | A1 | 21-11-2019 | CN | 112514541 A | 16-03-2021 |
| | | | CN | 114599144 A | 07-06-2022 |
| | | | CN | 114650646 A | 21-06-2022 |
| | | | EP | 3794912 A1 | 24-03-2021 |
| | | | EP | 4224998 A1 | 09-08-2023 |
| | | | EP | 4224999 A2 | 09-08-2023 |
| | | | JP | 6977212 B2 | 08-12-2021 |
| | | | JP | 2021518644 A | 02-08-2021 |
| | | | KR | 20210011419 A | 01-02-2021 |
| | | | RU | 2767304 C1 | 17-03-2022 |
| | | | US | 10367508 B1 | 30-07-2019 |
| | | | US | 2019357342 A1 | 21-11-2019 |
| | | | US | 2019357343 A1 | 21-11-2019 |
| | | | WO | 2019222627 A1 | 21-11-2019 |
| EP 1317944 | A1 | 11-06-2003 | AT | E343414 T1 | 15-11-2006 |
| | | | DE | 60124123 T2 | 06-09-2007 |
| | | | EP | 1317944 A1 | 11-06-2003 |
| | | | ES | 2275644 T3 | 16-06-2007 |

EPO FORM P0459

For more details about this annex : see Official Journal of the European Patent Office, No. 12/82